**Europäisches Patentamt**

**European Patent Office**

**Office européen des brevets**

(11) Veröffentlichungsnummer: **0 026 481**
**B1**

(12) **EUROPÄISCHE PATENTSCHRIFT**

(45) Veröffentlichungstag der Patentschrift:
**16.01.85**

(21) Anmeldenummer: **80105831.4**

(22) Anmeldetag: **25.09.80**

(51) Int. Cl.⁴: **A 61 N 1/36,** H 02 M 3/18,
H 03 K 5/01

(54) Herzschrittmacher.

(30) Priorität: **27.09.79 DE 2939233**

(43) Veröffentlichungstag der Anmeldung:
**08.04.81 Patentblatt 81/14**

(45) Bekanntmachung des Hinweises auf die Patenterteilung:
**16.01.85 Patentblatt 85/3**

(84) Benannte Vertragsstaaten:
**CH DE FR GB IT LI NL SE**

(56) Entgegenhaltungen:
**DE - A - 2 064 402**
**DE - A - 2 731 619**
**DE - A - 2 823 804**
**DE - A - 2 825 626**
**GB - A - 1 477 537**
**US - A - 4 050 004**
**US - A - 4 132 233**

(73) Patentinhaber: **Siemens Aktiengesellschaft, Berlin und München Wittelsbacherplatz 2, D-8000 München 2 (DE)**

(72) Erfinder: **Elmqvist, Hakan, Dr., Sunnerdahlsvägen 7, S-161 38 Bromma (SE)**

## Beschreibung

Die Erfindung bezieht sich auf einen Herzschrittmacher mit Schrittaktgeber und Formschaltung für die Reizimpulse nebst Batterie, wobei die Formschaltung Kondensatoren umfasst, die parallel ladbar und über Halbleiterschalter seriell entladbar sind.

Ein Herzschrittmacher dieser Art ist beispielsweise durch die US-PS 4 050 004 vorbekannt. Dieser Herzschrittmacher arbeitet mit einer Batteriespannung in Höhe von etwa 2,5 V. Mit insgesamt vier Kondensatoren ergibt sich bei Parallelbeschickung und serieller Entladung eine Ausgangsspannung von ca. 10 V. Eine solche Ausgangsspannung ist jedoch nicht optimal an die tatsächlichen Umgebungsbedingungen des Schrittmachers angepasst. Häufig ist es so, dass ein Schrittmacher gewünscht wird, dessen Ausgangsamplitude nach vorgebbarem Bedarf variierbar ist. Beispielsweise kann zum Zweck der Messung der Reizschwelle des schrittmacherunterstützten Herzens ein treppenförmiger Verlauf der Reizamplitude wünschenswert sein. Für andere Zwecke können u.U. andere Verläufe der Amplitude der Reizimpulse erstrebenswert sein. Insgesamt sollte jedoch der Herzschrittmacher energiemässig immer so ausgelegt sein, dass die Batterie nach Implantation so wenig wie nur möglich belastet wird, so dass sich für den implantierten Schrittmacher längere Lebensdauer ergibt. Auch deshalb sollte der Schrittmacher in der Ausgangsamplitude seiner Schrittmacherimpulse möglichst weitgehend variierbar sein.

Aufgabe der vorliegenden Erfindung ist es, einen Schrittmacher der eingangs genannten Art aufzubauen, der im Hinblick auf die Ausgangsamplitude der Reizimpulse grössere Variationsmöglichkeiten bietet als bisher.

Diese Aufgabe wird erfindungsgemäss dadurch gelöst, dass auch in den Ladestrecken der Kondensatoren Halbleiterschalter vorgesehen sind und nach Programmen eines Programmgebers einzelne Kondensatoren in beliebig nach Bedarf vorgebbaren Kombinationen zur parallelen Aufladung und anschliessenden seriellen Entladung zusammen- bzw. wieder auseinander schaltbar sind.

Der Herzschrittmacher gemäss der Erfindung ermöglicht Vorwahl des Amplitudenverlaufes der Herzschrittmacherpulse nach Programm. Er kann demnach von aussen immer rasch und optimal an die augenblicklichen Bedürfnisse angepasst werden.

Bei Herzschrittmachern besteht insgesamt gesehen auch der Wunsch nach besonders kompakter Bauweise, da dadurch der Schrittmacher in den Dimensionen insgesamt kleiner wird. In vorteilhafter Ausgestaltung der Erfindung ergeben sich hierbei optimale Verhältnisse, wenn die Kondensatoren und Halbleiterschalter Bestandteil einer Kombination aus einer niederfrequenzmässig und einer hochfrequenzmässig betriebenen Formschaltung sind. In einem solchen Falle besteht Variationsmöglichkeit in einem weiten Spannungsbereich (z.B. zwischen 2,5 V und 40 V) bei optimal aufeinander abgestimmtem Kondensatorvolumen. Bekanntlich weisen in niederfrequenzmässig betriebenen Formschaltungen die Kondensatoren relativ hohe Kapazität auf. Zur Realisierung dieser grossen Kapazitäten müssen entsprechend grossvolumige Kondensatoren eingesetzt werden. Eine hochfrequenzmässig betriebene Formschaltung kann hingegen mit kleineren Kapazitäten und damit mit weniger voluminösen Kondensatoren arbeiten. Ein gesundes Gemisch zwischen beiden Betriebsarten gewährleistet also optimal breiten Spannungsvariationsbereich bei optimal niedrigem Raumaufwand für Kondensatoren. Eine hochfrequenzmässig betriebene Formschaltung hat ferner auch den Vorteil kleiner Zeitkonstante der RC-Glieder. Sie eignet sich demnach besonders gut zur Erfassung von Herzaktionspotentialen über die Herzschrittmacherelektrode. Die erfassten Signale lassen sich als Erkennungssignale für spontane Herzreaktionen zur Inhibierung des Schrittmachers verwenden. Um einen Herzschrittmacher gemäss vorliegender Erfindung möglichst vielseitig einsetzen zu können, sollte auch weitgehende variable Umschaltmöglichkeit zwischen niederfrequenzmässigem und hochfrequenzmässigem Betrieb bzw. der Kombination zwischen beiden gewährleistet sein. Es sollten also Schalter vorgesehen sein, die eine Betriebsweise allein im niederfrequenten Bereich oder im hochfrequenten Bereich oder in der Kombination von beiden gewährleisten. Die Schaltungsanordnung sollte auch so sein, dass in einfacher Modifikation für jeweils eine oder beide Betriebsarten ein zweiter Ausgang geschaffen ist, der den Anschluss an eine zweite Herzschrittmacherelektrode ermöglicht. Es ist damit ein bifokaler Schrittmacher geschaffen, der die Stimulation beider Herzkammern, beispielsweise bei Bedarf im Vorhof und im Ventrikel, ermöglicht.

Weitere Vorteile und Einzelheiten der Erfindung ergeben sich aus der nachfolgenden Beschreibung eines Ausführungsbeispiels anhand der Zeichnung in Verbindung mit den Unteransprüchen.

Es zeigen:

Fig. 1 die Erfindung im Prinzipschaltbild,

Fig. 2 eine niederfrequenzmässig betriebene Formschaltung zum Einsatz in der Fig. 1,

Fig. 3 eine hochfrequenzmässig betriebene Formschaltung zum Einsatz in der Fig. 1,

Fig. 4 eine Modifikationsmöglichkeit der niederfrequenzmässig betreibbaren Formschaltung zur Umrüstung auf Bifokal-Betrieb,

Fig. 5 bis 7 zwei Modifikationsmöglichkeiten für die hochfrequenzmässig betreibbare Formschaltung zur Umrüstung auf Bifokal-Betrieb.

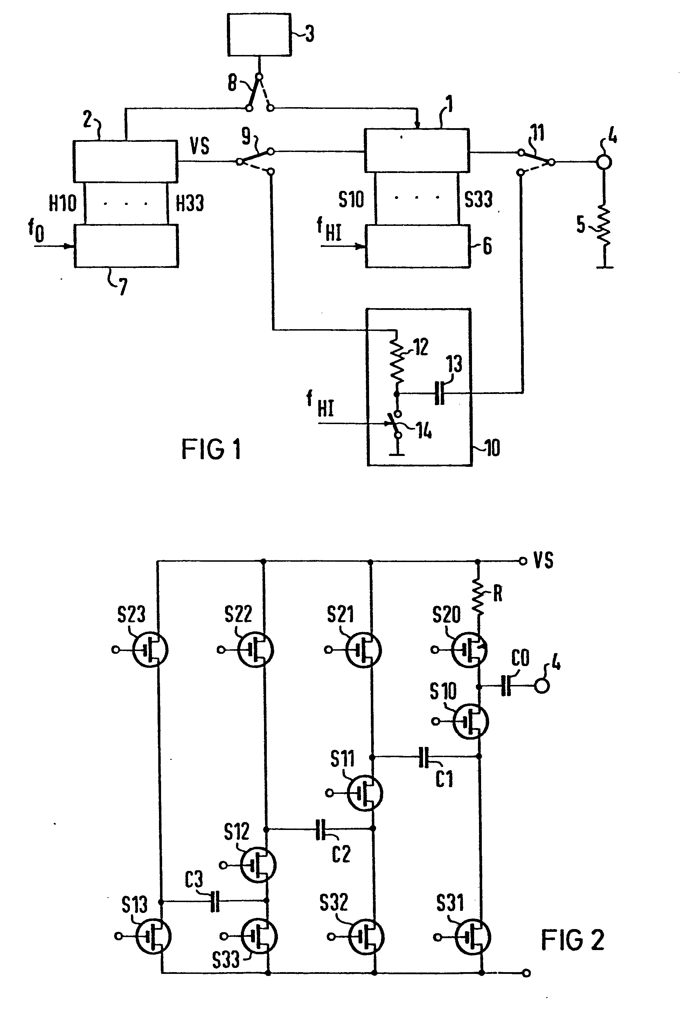

In der Fig. 1 ist eine niederfrequenzmässig zu betreibende Formschaltung mit 1 bezeichnet. Die Kennziffer 2 steht für eine hochfrequenzmässig zu betreibende Formschaltung. Die Batterie des Herzschrittmachers ist mit 3 angegeben. Die Herzschrittmacherelektrode ist mit 4 bezeichnet. Sie liegt am schematisch als Widerstand dargestellten menschlichen Herzen 5 an. Der niederfrequenzmässig zu betreibenden Formschaltung 1 ist ein Programmgeber 6 mit Programmausgängen S10 bis S33 zugeordnet. Entsprechend umfasst die hochfrequenzmässig zu betreibende Formschaltung 2 einen Programmgeber 7 mit den Programmausgängen H10 bis H33. Der Programmgeber 7 wird mit der Taktfrequenz $f_0 \approx 100$ Hz bis 10 kHz hochfrequenzmässig bedient. Die Steuerung des Programmgebers 6 erfolgt niederfrequenzmässig mit der Taktfrequenz der Schrittmacherimpulse $f_{HI}$ ($f_{HI} \approx 1$ Hz). Das Prinzipschaltbild der Fig. 1 erlaubt optimale Variation der Betriebsmöglichkeiten durch die Schalter 8, 9, 11 und 14. In der dargestellten Schaltstellung der einzelnen Schalter sind die beiden Formschaltungen für niederfrequenzmässigen und hochfrequenzmässigen Betrieb hintereinandergeschaltet. Am Ausgang ergibt sich somit Spannungsvariationsmöglichkeit zwischen z. B. 0 bis 40 V. Durch Umschaltung der Schalter 9 und 11 in die gestrichelte Schaltstellung erfolgt lediglich hochfrequenzmässiger Betrieb über das Schaltglied 10 mit RC-Glied 12, 13 und im Takt der Herzfrequenz $f_{HI}$ getaktetem Schalter 14. Bei Umschaltung des Schalters 8 in die gestrichelte Schaltstellung erfolgt lediglich niederfrequenzmässiger Betrieb. Durch Einschaltung lediglich einer der beiden Betriebsarten lässt sich die Spannung entsprechend in niedrigeren Bereichen variieren.

Eine Ausführungsform der niederfrequenzmässig zu betreibenden Formschaltung 1 zeigt die Fig. 2. Sie umfasst vier Kondensatoren C0 bis C3 mit zugehörigen Transistorschaltern S10 bis S33. Sämtliche Schalter S10 bis S33 sind einzeln oder in Kombination beliebig nach Programm des Programmgebers 6 in den leitenden Zustand steuerbar.

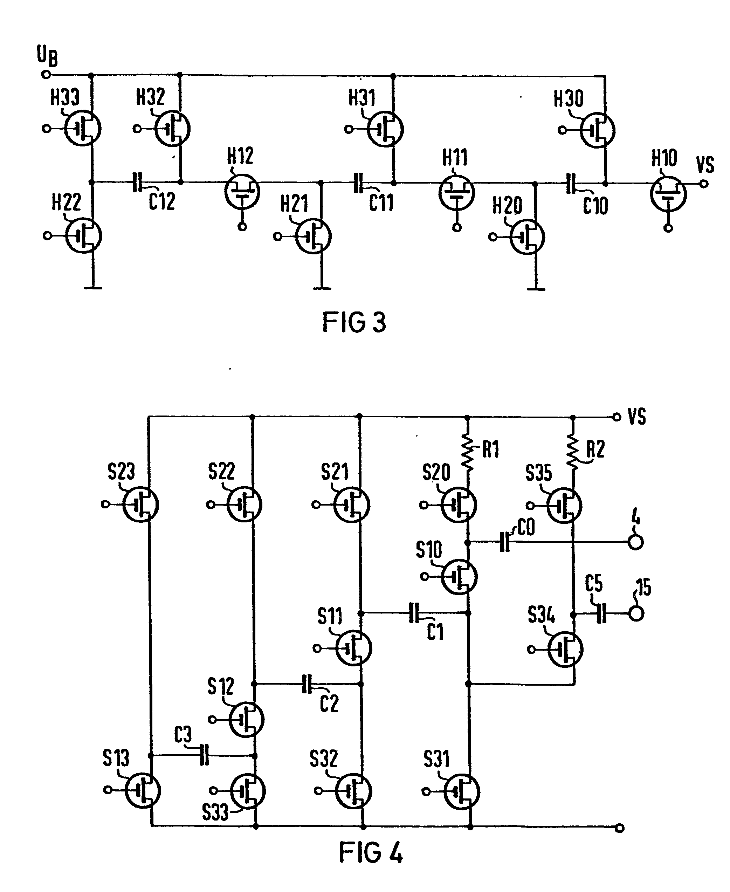

Ein Beispiel für eine hochfrequenzmässig zu betreibende Formschaltung 2 zeigt die Fig. 3. Diese Formschaltung weist die drei Kondensatoren C10 bis C12 auf. Die Schalter H10 bis H33 sind durch den Programmgeber 7 beliebig einzeln oder in Kombinationen betätigbar.

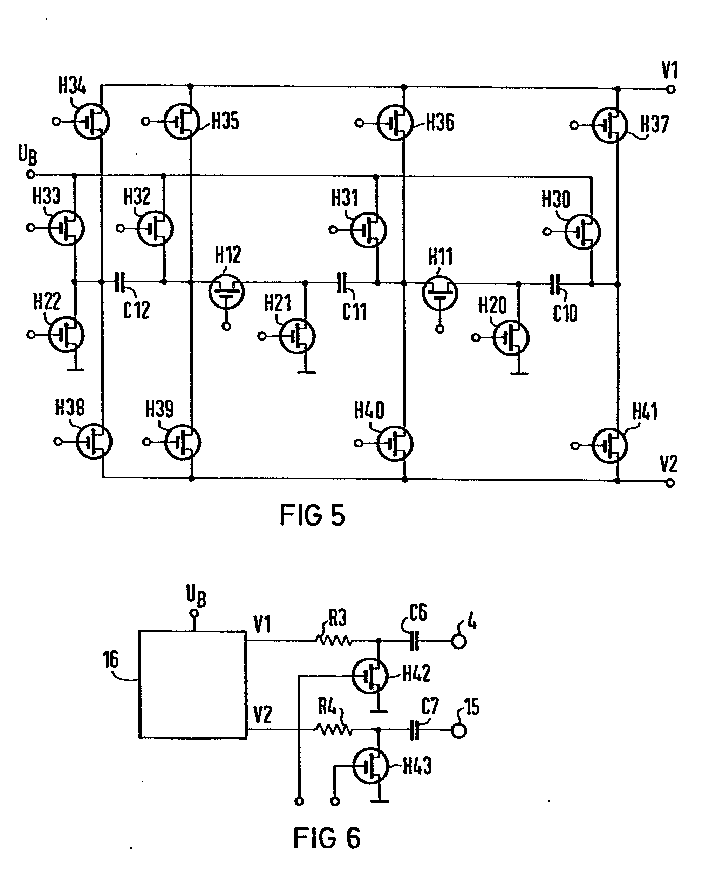

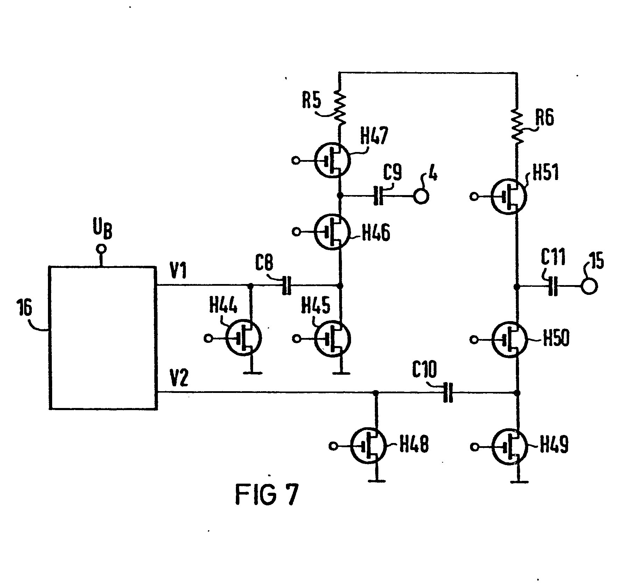

Die Fig. 4 bis 7 zeigen Modifikationen dieser beiden Ausführungsformen mit neuen Ausgangsstufen S34, S35, R2, C5 bzw. H34 bis H41 in Kombination mit H42, H43, R3, R4, C6, C7 oder H44 bis H51, C8 bis C11, R5, R6 in dem Sinne, dass ein zweiter Ausgang für einen zweiten Elektrodenanschluss 15 geschaffen wird. Hierdurch ergibt sich ein bifokaler Schrittmacher mit der Einsatzmöglichkeit zweier Reizelektroden, beispielsweise zur Stimulierung des Vorhofes und/oder des Ventrikels. In den Fig. 6 und 7 entspricht dabei der Block 16 dem Schaltbild der Fig. 5.

## Patentansprüche

1. Herzschrittmacher mit Schrittaktgeber und Formschaltung für die Reizimpulse nebst Betriebsspannungsgeber, insbesondere Batterie, wobei die Formschaltung Kondensatoren umfasst, die parallel ladbar und über Halbleiterschalter seriell entladbar sind, dadurch gekennzeichnet, dass auch in den Ladestrecken der Kondensatoren (C0 bis C3 bzw. C10 bis C12) Halbleiterschalter (S10 bis S33 bzw. H10 bis H33) vorgesehen sind, und nach Programmen eines Programmgebers (6 bzw. 7) einzelne Kondensatoren in beliebig nach Bedarf vorgebbaren Kombinationen zur parallelen Aufladung und anschliessenden seriellen Entladung zusammen- bzw. wieder auseinanderschaltbar sind.

2. Herzschrittmacher nach Anspruch 1, dadurch gekennzeichnet, dass Kondensatoren und Halbleiterschalter Bestandteil einer niederfrequenzmässig betriebenen Formschaltung (1) oder hochfrequenzmässig Formschaltung (2) oder Bestandteil einer Kombination aus einer niederfrequenzmässig und einer hochfrequenzmässig betriebenen Formschaltung (1 und 2) sind.

3. Herzschrittmacher nach Anspruch 2, dadurch gekennzeichnet, dass bei Einsatz einer niederfrequenzmässig und/oder hochfrequenzmässig betriebenen Formschaltung aus Kondensatoren und Halbleiterschaltern jeder Formschaltung (1, 2) ein entsprechender Programmgeber (6, 7) zugeordnet ist.

4. Herzschrittmacher nach einem der Ansprüche 1 bis 3, dadurch gekennzeichnet, dass in den Lade- bzw. Entladestrecken Abzapfstellen zum Anschluss einer zweiten Herzschrittmacherelektrode (15) vorgesehen sind.

5. Herzschrittmacher nach Anspruch 4, dadurch gekennzeichnet, dass im Falle des Einsatzes einer niederfrequenzmässig zu betreibenden Formschaltung ein Zusatzausgang für eine zweite Herzschrittmacherelektrode (15) durch eine Ausgangs-Parallelschaltung (S34, S35, R2, C5) geschaffen ist.

6. Herzschrittmacher nach Anspruch 4, dadurch gekennzeichnet, dass im Falle des Einsatzes einer hochfrequenzmässig zu betreibenden Formschaltung eine einzelne Ausgangsstufe vorgesehen ist oder anstelle der einzelnen Ausgangsstufe (H10, VS) für jeden Kondensator (C10 bis C12) eine Doppelserie von Halbleiterschaltern (H34 bis H37 bzw. H38 bis H41) vorhanden ist zur Schaffung zweier separater Spannungsgänge (V1, V2), denen für zwei separate Herzschrittmacherelektroden (4, 15) zwei weitere Ausgangsstufen (H42, H43, R3, R4, C6, C7 oder H44 bis H51, C8 bis C11, R5, R6) zugeordnet sind.

## Claims

A heart pacemaker having a pace pulse generator and shaping circuit for the stimulating pulses, together with an operating voltage source, in particular a battery, where the shaping circuit comprises capacitors which can be charged in parallel and discharged serially by means of semiconductor switches, characterised in that in addition semiconductor switches (S10 to S33 or H10 to H33 as the case may be) are arranged in the charge paths of the capacitors (C0 to C3 or C10 to C12 as the case may be), and in accordance with programmes of a programmer (6 or 7 as the case may be) individual capacitors can be interconnected and separated again in combinations predetermined as necessary, for the parallel charging or subsequent serial discharging, as the case may be.

2. A heart pacemaker as claimed in Claim 1, characterised in that capacitors and semiconductor switches form part of a shaping circuit (1) operating at low-frequency, or a shaping circuit (2) operating at high-frequency, or part of a combination of a low-frequency shaping circuit and a high-frequency shaping circuit (1 and 2).

3. A heart pacemaker as claimed in Claim 2, characterised in that when using a low-frequency shaping circuit and/or a high-frequency shaping circuit which consists of capacitors and semiconductor switches, each shaping circuit (1, 2) is assigned a corresponding programmer (6, 7).

4. A heart pacemaker as claimed in one of Claims 1 to 3, characterised in that tapping points for connecting a second heart pacemaker electrode (15) are arranged in the charging and discharging paths.

5. A heart pacemaker as claimed in Claim 4, characterised in that when a low-frequency shaping circuit is used an additional output for a second heart pacemaker electrode (15) is formed by a parallel output circuit (S34, S35, R2, C5).

6. A heart pacemaker as claimed in Claim 4, characterised in that when using a high-frequency shaping circuit there is provided an individual output stage or, in place of the individual output stage (C10, VS), a double series of semiconductor switches (H34 to H37 and H38 to H41) for each capacitor (C10 to C12) in order to form two separate voltage paths (V1, V2) which are assigned two further output stages (H42, H43, R3, R4, C6, C7 or H44 to H51, C8 to C11, R5, R6) for two separate heart pacemaker electrodes (4, 15).

**Revendications**

1. Stimulateur cardiaque comportant un générateur de cadence pas à pas et un circuit de mise en forme pour les impulsions d'excitation ainsi qu'un générateur d'une tension de service, notamment une pile, le circuit de mise en forme comportant des condensateurs qui peuvent être chargés en parallèle et déchargés en série par l'intermédiaire de commutateurs à semiconducteurs, caractérisé par le fait qu'il est également prévu des commutateurs à semi-conducteurs (S10 à S33 ou H10 à H33) dans les voies de charge des condensateurs (C0 à C3 ou C10 à C12), et que différents condensateurs peuvent être interconnectés ensemble ou à nouveau déconnectés les uns des autres, conformément aux programmes d'un générateur de programmes (6 ou 7), selon des combinaisons pouvant être prédéterminées à volonté en cas de besoin, pour réaliser la charge en parallèle et la décharge ultérieure en série.

2. Stimulateur cardiaque suivant la revendication 1, caractérisé par le fait que les condensateurs et les commutateurs à semiconducteurs font partie d'un circuit de mise en forme (1) fonctionnant à basse fréquence ou d'un circuit de mise en forme (2) fonctionnant à haute fréquence ou bien font partie d'une combinaison d'un circuit de mise en forme (1) fonctionnant à basse fréquence et d'un circuit de mise en forme (2) fonctionnant à haute fréquence.

3. Stimulateur cardiaque suivant la revendication 2, caractérisé par le fait que dans le cas de l'utilisation d'un circuit de mise en forme fonctionnant à basse fréquence et/ou d'un circuit de mise en forme fonctionnant à haute fréquence, constitué par des condensateurs et par des commutateurs à semiconducteurs, à chaque circuit de mise en forme (1) est associé un générateur correspondant de programmes (6, 7).

4. Stimulateur cardiaque suivant l'une des revendications 1 à 3, caractérisé par le fait que dans les voies de charge et de décharge il est prévu des prises servant au raccordement d'une seconde électrode (15) du stimulateur cardiaque.

5. Stimulateur cardiaque suivant la revendication 4, caractérisé par le fait que dans le cas de l'utilisation d'un circuit de mise en forme devant fonctionner à haute fréquence, une sortie supplémentaire pour une seconde électrode (15) du stimulateur cardiaque est formée par un circuit parallèle de sortie (S34, S35, R2, C5).

6. Stimulateur cardiaque suivant la revendication 4, caractérisé par le fait que dans le cas de l'utilisation d'un circuit de mise en forme devant fonctionner à haute fréquence, il est prévu un étage individuel de sortie ou bien, à la place de l'étage individuel de sortie (H10, VS), pour chaque condensateur (C10 à C12) il est prévu une série double de commutateurs à semiconducteurs (H34 à H37 ou H38 à H41), de manière à créer deux sorties séparées de tension (V1, V2) auxquelles sont associés, pour deux électrodes séparées (4, 15) du stimulateur cardiaque, deux étages supplémentaires de sortie (H42, H43, R3, R4, C6, C7 ou H44 à H51, C8 à C11, R5, R6).

FIG 1

FIG 2

FIG 3

FIG 4

FIG 5

FIG 6

FIG 7